**Europäisches Patentamt**

**European Patent Office**

**Office européen des brevets**

(19)

(11) Numéro de publication: **0 203 000
B1**

(12) **FASCICULE DE BREVET EUROPEEN**

(45) Date de publication du fascicule du brevet:
02.08.89

(51) Int. Cl.⁴: **C07D 309/00**

(21) Numéro de dépôt: **86401016.0**

(22) Date de dépôt: **13.05.86**

(54) Procédé et installation de cristallisation de gluconodeltalactone.

(30) Priorité: **15.05.85 FR 8507433**

(43) Date de publication de la demande:
**26.11.86 Bulletin 86/48**

(45) Mention de la délivrance du brevet:
**02.08.89 Bulletin 89/31**

(84) Etats contractants désignés:
**DE FR IT NL**

(56) Documents cités:
**FR-A- 2 552 777**

**CHEMICAL ABSTRACTS,
vol. 85, no. 1, 5 juillet 1976, page 488, résumé no. 6008k,
Columbus, Ohio, US**

(73) Titulaire: **Roquette Frères, F-62136 Lestrem(FR)**

(72) Inventeur: **Leleu, Jean Bernard, 22 place du 8 Mai,
F-62136 Lestrem(FR)**
Inventeur: **Lemay, Patrick, 29 Rue du Puits Richebourg,
F-62136 Lestrem(FR)**

(74) Mandataire: **Koch, Gustave et al, Cabinet
PLASSERAUD 84, rue d'Amsterdam, F-75009 Paris(FR)**

## Description

L'invention a pour objet un procédé et une installation de cristallisation de gluconodeltalactone ou GDL.

Il est connu, notamment par le brevet U. S. No. 2.102.380, de préparer de la gluconodeltalactone cristallisée à partir de solutions aqueuses riches en acide gluconique en présence de cristaux de GDL qui jouent le rôle de germes de cristallisation.

Il est également connu, par la demande de brevet japonais publiée No. 49-29312 du 13 mars 1974 au nom de DAI ICHI KOGYO SEIYAKU K. K. (Chemical Abstracts, vol. 85, résumé 6008 k), de procéder à cette opération dans un cristallisoir de concentration de type rotatif en respectant les conditions de température et de concentration du milieu soumis à cristallisation indiquées dans cette demande de brevet.

Ces procédés ne donnent pas entièrement satisfaction tant du point de vue de la productivité par unité de volume de l'appareillage que de celui du bilan énergétique.

On connait par EP-A 0 147 269 un procédé de cristallisation comportant un reyclage relatif au dextrose monohydrate. Pour faire face aux contraintes notamment économiques toujours plus sévères, la Société Demanderesse a cherché à mettre au point un procédé et une installation répondant mieux que ceux qui existent déjà aux divers desiderata de la pratique, en particulier précisément du point de vue de la productivité de l'opération de cristallisation par unité de volume de l'appareillage utilisé et du bilan énergétique.

Et elle a trouvé que ce but pouvait être atteint grâce à un procédé de cristallisation en continu de gluconodeltalactone ou GDL, caractérisé par le fait qu'un sirop d'acide gluconique de préférence exempt de cristaux et de nucléi, d'une richesse en acide gluconique supérieure à 80, de préférence supérieure à 90%, d'une concentration en matières sèches de 80 à 95%. de préférence de 85 à 90% en poids et d'une température de 60 à 85°C, de préférence de 65 à 75°C, est introduit dans une première zone de cristallisation d'axe de préférence sensiblement vertical qu'il est amené à parcourir sous agitation et à l'intérieur de laquelle il est maintenu à une température sensiblement constante, inférieure de 2 à 10°C et de préférence de 3 à 5°C à la température de saturation, ce grâce à quoi se produit l'amorçage de la cristallisation qui se traduit par la formation d'un mélange de sirop et de cristaux de GDL, la durée de séjour moyen d'une fraction donnée de mélange à l'intérieur de la zone étant de 10 à 30 heures, de préférence de 15 à 25 heures, de façon telle que ce mélange sortant de la zone présente une concentration en cristaux de 2 à 15%, de préférence de 3 à 10%, ledit mélange sortant de la première zone étant amené à parcourir, de haut en bas, sous malaxage, une deuxième zone de cristallisation d'axe de préférence sensiblement vertical disposé de préférence sensiblement dans le prolongement de celui de la première zone, un gradient de température globalement décroissant éventuellement modulé de 0,2 à 1°C/heure, de préférence de 0,3 à 0,6°C/heure étant imposé à l'intérieur de la deuxième zone au mélange qui la traverse, ce gradient étant de préférence limité à une première partie de cette deuxième zone, cette première partie s'étendant depuis l'extrémité supérieure de la deuxième zone jusqu'à un niveau de celle-ci situé entre sa mihauteur et le tiers inférieur de sa hauteur totale, cette première partie de la deuxième zone étant suivie d'une seconde partie jouant le rôle de zone mûrissement, située dans le prolongement de la première et à l'intérieur de laquelle la température est maintenue constante entre 35 et 50°C, de préférence entre 40 et 45°C, le mélange sortant de la deuxième partie de la deuxième zone sous la forme d'une masse cristallisée riche en cristaux et à partir de laquelle on récupère ces derniers, l'amorçage de la cristallisation au niveau de la première zone étant favorisé par le recyclage de préférence au niveau de l'extrémité supérieure de celle-ci d'une fraction du mélange parcourant la seconde zone ou, lorsqu'elle est prévue, la première partie de cette seconde zone, cette fraction recyclée représentant de 10 à 120%, de préférence de 40 à 110% et plus préférentiellement encore de 80 à 100%, de la quantité de sirop introduite dans la première zone, cette fraction, qui est prélevée à un niveau situé dans la moitié inférieure de la seconde zone ou, lorsqu'elle est prévue, de la moitié inférieure de la première partie de la seconde zone, étant avantageusement soumise à une fragmentation des cristaux qu'elle contient avant son introduction au niveau de l'extrémité supérieure de la première zone.

Pour mettre en oeuvre le susdit procédé, on peut avoir recours, conformément à l'invention, à une installation comprenant essentiellement deux enceintes d'axes de préférence sensiblement verticaux disposées de préférence l'une au-dessus de l'autre, les axes des deux enceintes étant de préférence sensiblement dans le prolongement l'un de l'autre,

- la première enceinte, ou enceinte d'amorçage de la cristallisation, étant équipée d'une part d'un système d'alimentation en sirop riche en acide gluconique de préférence au voisinage de son extrémité supérieure, d'autre part d'un système d'agitation du contenu de l'enceinte et d'un système de régulation de la température propre à établir à l'intérieur de l'enceinte une température sensiblement constant en tous points en enfin d'un système d'extraction disposé au voisinage de son extrémité inférieure, ce système étant propre à extraire le mélange de sirop et de cristaux formé à l'intérieur de l'enceinte et à acheminer ce mélange à un point situé an voisinage de l'extrémité supérieure de

-la deuxième enceinte, ou de cristallisation proprement dite et équipée d'un système de malaxage du contenu et d'un système de régulation de la température propre à établir au sein de la masse soumise à cristallisation qui la remplit, un gradient de température globalement décroissant de haut en bas et avantageusement limité à une première partie de la deuxième enceinte, cette première partie s'étendant depuis l'extrémité supérieure de l'enceinte jusqu'à un niveau situé entre la mi-hauteur et le tiers inférieur de sa hauteur totale, ledit système de régulation de température étant alors propre à établir, dans une seconde partie de cette deuxième enceinte, une température globalement constante voisine de celle régnant au niveau de l'extrémité inférieure du susdit gradient, cette seconde partie faisant suite vers le bas à la première partie, ladite deuxième enceinte étant par ailleurs équipée, au voisinage de son extrémité inférieure, d'un système d'extraction en continu d'un produit fortement enrichi en cristaux de GDL qui est acheminé par des moyens appropriés vers un système adapté à récupérer les cristaux de GDL à partir de ce produit, ladite installation étant en outre équipée d'un système de recyclage vers un point situé de préférence au voisinage de l'extrémité supérieure de la première enceinte d'une partie de contenu de la deuxième enceinte prélevé à un niveau situé dans la moitié inférieure de la deuxième enceinte, le cas échéant dans la moitié inférieure de la première partie de la deuxième enceinte, ledit système de recyclage comprenant avantageusement des moyens de fractionnement des cristaux contenus dans la masse recyclée.

L'invention vise également d'autres dispositions qui s'utilisent de préférence en même temps et dont il sera plus explicitement question ci-après.

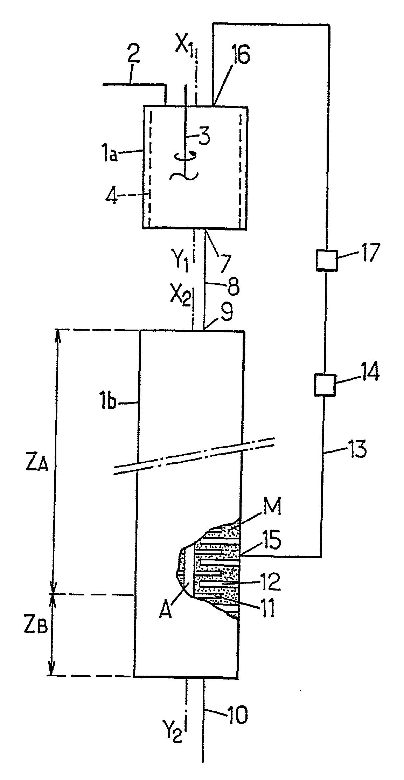

Et elle purra, de toute façon, être bien comprise à laide du complément de description qui suit et du dessin annexé qui sont relatifs à des modes de réalisation avantageux.

La figure unique du dessin montre schématiquement une installation conforme à l'invention.

Se proposant, par conséquent, de préparer de la gluconodeltalactone cristallisée conformément à l'invention, on s'y prend comme suit ou de façon équivalente.

On utilise comme matière première un sirop riche en acide gluconique, de préférence exempt de cristaux et de nucléi, provenant par exemple de la fermentation oxydative du glucose ; ce sirop présente une teneur en matières sèches d'environ 80 à 95%, de préférence de 85 à 90% en poids, l'acide gluconique entrant pour au moins 80% et, de préférence, pour une proportion supérieure à 90% en poids sur matières sèches dans sa constitution.

Ce sirop concentré est acheminé vers une première zone de cristallisation d'axe de préférence sensiblement vertical, qu'il est amené à parcourir en continu sous agitation et à l'intérieur de laquelle il est maintenu à une température sensiblement constante, inférieure à la température de saturation, notamment de 2 à 10°C et de préférence de 3 à 5°C, ce grâce à quoi se produit l'amorçage de la cristallisation qui se traduit par la formation d'un mélange de siròp et de cristaux de GDL.

La durée de séjour moyen d'une fraction donnée de mélange à l'intérieur de cette première zone est de 10 à 30 heures et de préférence de 15 à 25 heures, de telle sorte que le mélange sortant de l'enceinte présente une concentration en cristaux de 2 à 15%, de préférence de 3 à 10%.

Le mélange sortant de la première zone est amené ensuite, à traverser de haut en bas, sous malaxage, une deuxième zone de cristallisation d'axe de préférence sensiblement vertical, disposé de préférence dans le prolongement de celui de la première zone.

La température du mélange est maintenue, de préférence, au moment de son introduction dans la deuxième zone de cristallisation, à une valeur proche de celle qui régne à l'intérieur de la première zone.

Un gradient de température globalement décroissant de haut en bas de 0,2°c à 1°C/heure, de préférence de 0,3 à 0,6°C/heure, est imposé au mélange, c'est-à-dire à la masse soumise à la cristallisation qui traverse cette zone; de préférence, ce gradient est limité à une première partie de la deuxième zone de cristallisation ; cette première partie de la deuxième zone de cristallisation s'étend de l'extrèmité supérieure de la deuxième zone jusqu'à un niveau de celle-ci situé entre sa mi-hauteur et le tiers inférieur de sa hauteur totale.

La première partie de la deuxième zone est suivie d'une seconde partie située de préférence dans le prolongement de la première et à l'intérieur de laquelle la température est maintenue constante, de préférence à une valeur de 35 à 50°C, de préférence de 40 à 45 °C. Cette seconde partie joue en fait le rôle d'une zone de mûrissement.

Le mélange sortant de cette seconde partie se trouve sous la forme d'une masse cristallisée riche en cristaux de GDL, à partir de laquelle on récupère ces derniers.

La richesse de cette masse en cristaux de GDL est de 30 à 60%, de préférence de 40 à 55%.

L'ensemble de la masse remplissant la deuxième zone de cristallisation, c'est-à-dire de préférence les première et seconde parties de celle-ci, parcourt cette zone à la façon d'un "piston", terme utilisé dans la technique.

L'amorçage de la cristallisation au niveau de la première zone est favorisé par le recyclage de préférence au niveau de l'extrémité supérieure de celle-ci d'une fraction du mélange parcourant la deuxième zone et, lorsqu'elle est prévue, la première partie de la seconde zone, cette fraction recyclée représentant de 10 à 120% et, de préférence, de 40 à 110% et plus préférentiellement encore de 80 à 100% du sirop introduit dans la première zone.

Cette fraction est prélevée à un niveau situé dans la partie inférieure de la deuxième zone et, lorsqu'elle est prévue, dans la partie inférieure de la première partie de la deuxième zone ; ceci correspond dans la pratique à recycler une masse dont la température est inférieure d'environ 7,5 à 15°C à celle régnant dans la première zone.

La fraction recyclée est de plus soumise, de préférence, à un fractionnement des cristaux qu'elle contient avant son introduction de préférence au niveau de l'extrémité supérieure de la première zone.

Grâce au procédé conforme à l'invention, on extrait en continu, au voisinage de l'extrémité inférieure de la deuxième zone ou, lorsqu'elle est prévue, de la deuxième partie de la zone de cristallisation proprement dite, une masse riche en cristaux de GDL sans qu'il se produise de dérèglement des paramètres du processus de cristallisation, dérèglement qui se répercuterait au niveau de l'étape suivante de séparation de la phase liquide et des cristaux et qui pourrait nécessiter des arrêts intermittents de l'installation. En d'autres termes, ce porocédé permet d'arriver à une productivité très favorable par unité de volume de l'appareillage utilisé pour la mise en oeuvre du procédé.

Cette productivité est supérieure à celle obtenue dans les procédés de l'art antérieur.

Le débit d'alimentation en sirop riche en acide gluconique est choisi de façon telle que le temps de séjour moyen, d'une fraction donnée de la masse soumise à cristallisation à l'intérieur de la deuxième zone de cristallisation est de 40 à 80 heures, de préférence de 60 à 70 heures ; la valeur adoptée dépend des capacités d'échange thermique des moyens comportés par cette deuxième zone et à l'aide desquels est établi, à l'intérieur de cette seconde zone, de préférence à l'intérieur d'une première partie de ladite zone au sein de la masse soumise à la cristallisation, le gradient de température décroissant dont il a été question plus haut.

La viscosité de la masse soumise à cristallisation qui augmente au fur et à mesure que croît la proportion de cristaux de GDL, c'est-à-dire dans le sens descendant, fait que la zone de cristallisation est, de préférence, équipée de moyens de refoulement ou d'aspiration propres à faciliter le cheminement de la masse à l'intérieur de la zone.

Par ailleurs, les moyens de malaxage et d'homogénéisation comportés par la deuxième zone de cristallisation doivent être agencés de telle sorte que les zones mortes soient évitées et que l'échange thermique entre la masse soumise à cristallisation et les moyens de refroidissement soit le plus efficace possible.

Le produit extrait de la deuxième zone de cristallisation et qui constitue, comme déjà indiqué, une masse riche en cristaux de GDL, comprend des cristaux de GDL d'un spectre granulométrique caractérisé par une faible proportion de fins et de gros cristaux et donc par une forte proportion de cristaux de taille intermédiaire, ce spectre ne variant pas dans le temps, ce grâce à quoi l'étape de traitement suivante, qui consiste à séparer ces cristaux de la phase liquide dans laquelle ils baignent, ne connaît pas de perturbation.

Cette séparation comprend un turbinage et éventuellement un lavage grâce auxquels on récupère la majeure partie de la phase liquide : celle-ci forme des eaux-mères dont la concentration en acide gluconique est inférieure à celle du sirop riche en acide gluconique de départ -- cette concentration atteint généralement de 75 à 98% et plus généralement de 80 à 92% -- et dans lesquelles on retrouve la presque totalité des impuretés contenues dans ledit sirop de départ.

Les eaux-mères recueillies peuvent être partiellement recyclées et entrer alors dans la constitution du sirop d'alimentation.

Ceci étant, pour mettre en oeuvre le procédé conforme à l'invention, on peut avoir recours à l'installation dont il va être question à présent.

Cette installation comprend essentiellement deux enceintes 1a et 1b avantageusement disposées l'une (1a) au-dessus de l'autre (1b) ; ces enceintes ont avantageusement la forme de cylindres de révolution d'axes $X_1$, $Y_1$ et $X_2$, $Y_2$ de préférence sensiblement verticaux et de préférence situés dans le prolongement l'un de l'autre.

L'enceinte 1a est équipée

-d'un système d'alimentation en sirop riche en acide gluconique au niveau de son extrémité supérieure et représenté schématiquement par une canalisation 2,

-d'un système d'agitation 3 et

-d'un système de régulation de la température schématiquement représenté en 4 et propre à établir une température constante en tous points à l'intérieur de l'enceinte.

Le mélange constitué de sirop d'acide gluconique et de cristaux de GDL qui s'est formé à l'intérieur de l'enceinte de l'extrémité inférieure de cette enceinte; à cet endroit l'enceinte peut comporter une canalisation 8 par laquelle le mélange est acheminé vers l'enceinte 1b ; on peut également prévoir que l'orifice de sortie de l'enceinte 1a soit disposé en regard de l'orifice d'entrée de l'enceinte 1b, les deux enceintes étant alors juxtaposées.

En règle générale, c'est toutefois la disposition représentée sur la figure qui est adoptée, les deux enceintes étant disposées l'une sous l'autre de préférence dans le prolongement l'une de l'autre, la canalisation 8 jouant simultanément le rôle de canalisation d'extraction pour l'enceinte 1a et de canalisation d'alimentation en mélange de sirop d'acide gluconique et de cristaux de GDL pour l'enceinte 1b en un point 9 de celle-ci voisin de son extrémité supérieure.

L'enceinte 1b est équipée

- d'un système de malaxage et de régulation de la température dont il va être question et

- d'un système d'extraction en continu au niveau de l'extrémité inférieure de l'enceinte et schématiquement représenté par une canalisation 10, ce système étant propre à récupérer la masse riche en cristaux de GDL obtenue à la sortie de l'enceinte 1b.

Le système de malaxage et de régulation de la température dont il est question ci-dessus peut avantageusement comporter

- un ensemble de bras de malaxage 11 portés à intervalles réguliers par un arbre rotatif A dont l'axe est confondu avec l'axe $X_2Y_2$ de l'enceinte 1b,

- des nappes de refroidissement 12 disposées en alternance avec les bras malaxeurs 11 et portées par la paroi de l'enceinte 1b, ces nappes de refroidissement étant parcourues par un fluide de refroidissement.

Le système de régulation de la température est agencé de telle sorte qu'il permette d'établir à l'intérieur de l'enceinte 1b, un gradient de température globalement décroissant de haut en bas.

De préférence et comme prévue dans le mode de réalisation décrit, ledit système est agencé de telle sorte qu'il établisse à l'intérieur de l'enceinte 1b,

- une première partie $Z_A$ partant de l'extrémité supérieure de l'enceinte et à l'intérieur de laquelle est imposé à la masse contenue dans l'enceinte un gradient de température globalement décroissant vers le bas,
- une deuxième partie $Z_B$ située au-dessous et dans le prolongement de $Z_A$ et à l'intérieur de laquelle est imposée à la masse contenue dans l'enceinte une température sensiblement constante en tous points.

La première partie $Z_A$ représente de 1/2 à 2/3 de la longueur totale de l'enceinte 1b.

L'enceinte 1b comporte en outre des moyens globalement représentés par une canalisation 13 comportant une pompe 14 et propres

- à prélever à un niveau 15 situé dans la moitié inférieure de la première partie $Z_A$de l'enceinte, une fraction de la masse M soumise à cristallisation et parcourant l'enceinte 1b de haut en bas et
- à recycler cette fraction à un niveau 16 situé de préférence au voisinage de l'extrémité supérieure de l'enceinte 1a.

De préfèrence, la canalisation 13 comporte des moyens de fragmentation 17, par exemple un broyeur, propres à désagréger d'éventuels agrégats de cristaux d'acide gluconique contenus dans la fraction recyclée.

Pour l'établissement des deux parties $Z_A$ et $Z_B$, on fait intervenir non seulement la capacité d'échange thermique du système de régulation de température, mais également la vitesse de rotation des moyens de malaxage et la vitesse avec laquelle, sous l'influence des moyens d'aspiration non représentés, la masse soumise à cristallisation parcourt l'enceinte ; en d'autres termes, on fait intervenir la durée moyenne de séjour d'une fraction donnée de cette masse à l'intérieur de l'enceinte.

On signale que, dans la pratique, le fluide de refroidissement est de l'eau et que l'écart moyen de température en un point donné de l'enceinte entre cette eau et la masse soumise à cristallisation, est de l'ordre de 2 à 10°C.

## EXEMPLE

On a recours à une installation conforme à l'invention comportant deux enceintes cylindriques 1a et 1b de volumes utiles respectifs de 1 et de 3, 3 m³.

On introduit dans l'enceinte 1a, avec un débit de 50 1 par heure, un sirop d'acide gluconique ayant une teneur en matières sèches de 88% et comprenant 92% en poids sur matières sèches d'acide gluconique, les 8% restants étant constitués notamment par d'autres acides organiques.

La température du sirop à l'entrée de l'enceinte 1a est d'environ 65°C ; elle est de 61°C à l'intérieur de l'enceinte.

La durée de séjour moyen à l'intérieur de l'enceinte 1a d'une fraction donnée du mélange de sirop et de cristaux de GDL est d'environ 20 heures.

A la sortie de l'enceinte, ce mélange présente une concentration en cristaux de l'ordre de 7%.

Le mélange sortant de l'enceinte 1a est amené par la canalisation 8 en un point 9 de l'enceinte 1b situé au voisinage de l'extrémité supérieure de celle-ci.

A l'intérieur de l'enceinte 1b, ce mélange est soumis dans une partie $Z_A$ à un gradient de température globalement décroissant de 0,4°C/heure ; la température supérieure de ce gradient est de 60°C et la température inférieure, atteinte au niveau de l'extrémité inférieure de la partie $Z_A$ est de 45°C.

A l'intérieur de la partie $Z_B$qui fait suite à la partie $Z_A$, le mélange enrichi en cristaux de GDL est maintenu à la température de 45°C.

La partie $Z_B$ est parcourue en 30 heures environ.

Au niveau du point 15 situé à un niveau de la partie $Z_A$ correspondant à 50°C, c'est-à-dire à un point situé dans le tiers inférieur de cette zone, on prélève dans l'enceinte une fraction de la masse soumise à cristallisation qui la parcourt et on recycle cette fraction à l'extrémité supérieure de l'enceinte 1a en 16 après l'avoir soumise à l'action du broyeur.

La fraction recyclée correspond à 80% de la quantité de sirop introduite par la canalisation 2.

La masse riche en cristaux de GDL extraite au niveau de l'extrémité inférieure de l'enceinte 1b par la canalisation 10 se trouve à une température voisine de 45°C et permet de séparer une quantité de cristaux correspondant en poids à 43% du mélange.

La séparation des cristaux de GDL est effectuée par turbinage, puis les cristaux sont lavés.

La teneur en acide gluconique des eaux-mères ainsi récupérées après lavage des cristaux, est de 86%.

Le rendement de cristallisation qui est donné par la formule :

$$r = \frac{A - H}{100 - H}$$

dans laquelle

- A, qui représente la richesse en acide gluconique du sirop d'alimentation, est de 92%,
- H, qui représente la richesse des eaux-mères après lavage des cristaux, est de 86%,
s'établit à 43%.

On produit ainsi par jour 658 kg de GDL, ce qui correspond à une productivité de 200 kg par jour et par m³ de l'enceinte de cristallisation au lieu de 130 kg avec la méthode classique.

De plus, il ne se produit aucune perturbation nécessitant l'arrêt de l'installation qui fonctionne en continu.

Les cristaux recueillis après turbinage et lavage présentent d'excellentes propriétés physiques et chimiques.

Ces cristaux sont d'une pureté de 99,8%, leur indice d'écoulement est bon et leur répartition granulométrique est la suivante :

- cristaux de taille supérieure
à 250 µm ................... 25%

- cristaux de taille comprise
entre 100 et 350 μm ........ 92%
b) La même installation et les mêmes conditions opérationnelles sont utilisées.

Cependant, à un moment donné, après avoir atteint l'équilibre du système, la fraction recyclée est prélevée à un niveau situé à l'extérieur du domaine conforme à l'invention.

On constate alors rapidement une évolution des paramètres de cristallisation qui se manifeste après quelques heures par une mauvaise séparation au niveau du turbinage et qui finit par nécessiter l'arrêt de l'installation et l'enlèvement de la masse qui la remplit avant de la remettre en marche sous les conditions conformes à l'invention.

Comme il va de soi et comme il résulte d'ailleurs déjà de ce qui précède, l'invention ne se limite nullement à ceux de ses modes d'application et de réalisation qui ont été plus particulièrement envisagés ; elle en embrasse, au contraire, toutes les variantes, notamment celle où l'installation conforme à l'invention comporte une seule enceinte à l'intérieur de laquelle on matérialise, à l'aide de moyens appropriés, les zones d'amorçage de cristallisation, de cristallisation proprement dit et de mûrissement et celle où l'installation conforme à l'invention comporte les enceintes d'amorçage de cristallisation et de cristallisation proprement dites qui ont été décrites dans ce qui précède, la zone de mûrissement étant matérialisée par une troisième enceinte indépendante des deux autres, située de préférence dans leur prolongement et comportant des moyens propres à imposer une température constante à la masse qui la traverse et qui provient de la deuxième enceinte.

## Revendications

1. Procédé de cristallisation en continu du gluconodeltalactone caractérisé par le fait qu'un sirop d'acide gluconique d'une richesse en acide gluconique supérieure à 80%, d'une concentration en matières sèches de 80 à 95%, de préférence de 85 à 90% en poids et d'une température de 60 à 85°C, de préférence de 65 à 75°C, est introduit dans une première zone de cristallisation d'axe de préférence sensiblement vertical qu'il est amené à parcourir sous agitation et à l'intérieur de laquelle il est maintenu à une température sensiblement constante, inférieure de 2 à 10°C, de préférence de 3 à 5°C à la température de saturation, ce grâce à quoi se produit l'amorçage de la cristallisation qui se traduit par la formation d'un mélange de sirop et de cristaux de GDL, la durée de séjour moyen d'une fraction donnée de mélange à l'intérieur de la zone étant de 10 à 30 heures, de préférence de 15 à 25 heures, de façon telle que ce mélange sortant de la zone présente une concentration en cristaux de 2 à 15%, ledit mélange sortant de la première zone étant amené à parcourir, de haut en bas, sous malaxage, une deuxième zone de cristallisation d'axe de préférence sensiblement vertical, un gradient de température globalement décroissant éventuellement modulé de 0,2 à 1°C/heure étant imposé à l'intérieur de la deuxième zone au mélange qui la traverse, ce gradient étant de préférence limité à une première partie de cette deuxième zone, cette première partie d'étendant depuis l'extrémité supérieure de la deuxième zone jusqu'à un niveau de celle-ci situé entre sa mi-hauteur et le tiers inférieur de sa hauteur totale, cette première partie de la deuxième zone étant suivie d'une seconde partie jouant le rôle de zone de mûrissement, située dans le prolongement de la première et à l'intérieur de laquelle la température est maintenue constante entre 35 et 50°C, de préférence entre 40 et 45°C, le mélange sortant de la deuxième partie de la deuxième zone sous la forme d'une masse cristallisée riche en cristaux et à partir de laquelle on récupère ces derniers, l'amorçage de la cristallisation au niveau de la première zone étant favorisé par le recyclage au niveau de celle-ci d'une fraction du mélange parcourant la seconde zone ou la première partie de la seconde zone, cette fraction recyclée représentant de 10 à 120% de la quantité de sirop introduite dans la première zone, cette fraction étant prélevée à un niveau situé dans la moité inférieure de la seconde zone ou dans la moitié inférieure de la première partie de la seconde zone.

2. Procédé selon la revendication 1, caractérisé par le fait que le sirop d'acide gluconique mis en oeuvre est exempt de cristaux et de nucléi.

3. Procédé selon l'une des revendications 1 et 2, caractérisé par le fait que la richesse en acide gluconique du sirop mis en oeuvre est supérieure à 90% en poids.

4. Procédé selon l'une des revendications 1 à 3, caractérisé par le fait que le mélange sortant de la première zone présente une concentration en cristaux de 3 à 10% en poids.

5. Procédé selon l'une des revendications 1 à 4, caractérisé par le fait que l'axe de la deuxième zone est situé dans le prolongement de celui de la première zone.

6. Procédé selon l'une des revendications 1 à 5, caractérisé par le fait que le gradient de température est globalement décroissant est de 0,3 à 0,6°C/heure.

7. Procédé selon l'une des revendications 1 à 6, caractérisé par le fait que la fraction recyclée représente de 40 à 110%, de préférence de 80 à 100% de la quantité de sirop introduite dans la première zone.

8. Procédé selon l'une des revendications 1 à 7, caractérisé par le fait que la fraction recyclée est soumise à une fragmentation des cristaux qu'elle contient avant son introduction au niveau de l'extrémité supérieure de la première zone.

9. Installation pour la cristallisation en continu de gluconodeltalactone caractérisé par le fait qu'elle comprend essentiellement deux enceintes d'axes de préférence sensiblement verticaux disposées de préférence l'une au-dessus de l'autre, les axes des deux enceintes étant de préférence sensiblement dans les prolongement l'un de l'autre,
    - la première enceinte, ou enceinte d'amorçage de la cristallisation, étant équipée d'une part d'un système d'alimentation en sirop en acide gluconique au voisinage de son extrémité supérieure, d'autre part d'un système, d'agitation du contenu de l'enceinte et d'un système de régulation de la température propre à établir à l'intérieur de l'enceinte une température sensiblement constante en tous points et enfin d'un système d'extraction disposé au voisinage de son etrémité inférieure, ce système étant propre à extraire le mélange de sirop et de cristaux formé à l'intérieur de l'enceinte et à acheminer ce mélange à un point situé au voisinage de l'extrémité supérieure de -la deuxième enceinte, ou de cristallisation proprement dite et équipée d'un système de malaxage du contenu et d'un système de régulation de la température propre à établir au sein de la masse soumise à cristallisation qui la remplit, un gradient de température globalement décroissant de haut en bas depuis l'extrémité supérieure de l'enceinte, ce gradient étant limité de préférence à une première partie de cette deuxième enceinte qui s'étend jusqu'à un niveau situé entre la mi-hauteur et le tiers inférieur de la hauteur totale de l'enceinte, ledit système de régulation de la température établissant dans la seconde partie de cette deuxième enceinte, une température globalement constante coisine de celle régnant au niveau de l'extrémité inférieure du susdit gradient, cette seconde partie qui constitue une zone de mûrissement faisant suite vers le bas à la première partie, ladite deuxième enceinte étant par ailleurs équipée, au voisinage de son extrémité inférieure, d'un système d'extraction en continu d'un produit fortement enrichi en cristaux de GDL qui est acheminé par des moyens appropriés vers un système adapté à récupérer les cristaux de GDL à partir de ce produit, ladite installation étant en outre équipée d'un système de recyclage vers un point situé de préérence au voisinage de l'extrémité supérieure de la première enceinte d'une partie du contenu de la deuxième enceinte prélevé à un niveau situé dans la moitie inférieure de la deuxième enceinte ou, lorsqu'elle est prévue, dans la moitié inférieure de la première partie de la deuxième enceinte, ledit système de recyclage comprenant avantageusement des moyens de fractionnement des cristaux contenus dans la masse recyclée.

10. Installation selon la revendication 9, caractérisé par le fait qu'elle comporte une seule enceinte à l'intérieur de laquelle on matérialise, à l'aide de moyens appropriés, les zones d'amorçage de cristallisation, de cristallisation proprement dite et de mûrissement.

11. Installation selon la revendication 9, caractérisé par le fait qu'elle comporte, outre les première et deuxième enceintes, une troisiéme enceinte matérialisant la zone de mûrissement, située de préférence dans le prolongement des deux autres enceintes et comportant des moyens propres à imposer une température constante à la masse qui la traverse et qui provient de la deuxième enceinte.

**Patentansprüche**

1. Verfahren zur kontinuierlichen Kristallisation von Gluconcodeltalacton, dadurch gekennzeichnet, daß man einen Gluconsäuresirup mit einem Gehalt an Gloconsäure von höher als 80%, einer Trockensubstanzkonzentration von 80 bis 95%, vorzugsweise 85 bis 90%, auf das Gewicht bezogen, und mit einer Temperatur von 60 bis 85°C, vorzugsweise 65 bis 75°C, in eine erste Kristallisationszone mit vorzugsweise im wesentlichen vertikaler Achse einbringt, die man von dem Gluconsäuresirup unter Rühren durchlaufen läßt und in deren Innerem dieser auf einer im wesentlichen konstanten Temperatur von 2 bis 10°C, vorzugsweise 3 bis 5°C, unterhalb der Sättigungstemperatur gehalten wird, aufgrund dessen der Beginn der Kristallisation eintritt, der sich in einer Bildung eines Gemisches von Sirup und GDL-Kristallen äußert, wobei die durchschnittliche Verweildauer einer gegebenen Fraktion des Gemisches im Innern der Zone 10 bis 30 h, vorzugsweise 15 bis 25 h beträgt, derart, daß das die Zone verlassende Gemisch eine Konzentration an Kristallen von 2 bis 15% aufweist, das die erste Zone verlassende Gemisch von oben nach unten unter Durchkneten eine zweite Kristallisationszone mit einer vorzugsweise im wesentlichen vertikalen Achse durchlaufen läßt, ein insgesamt abnehmender gegebenenfalls modulierter Temperaturgradient von 0,2 bis 1°C/h im Inneren der zweiten Zone dem sie durchlaufenden Gemisch auferlegt wird, wobei dieser Gradient vorzugsweise auf einen ersten Teil dieser zweiten Zone begrenzt ist, dieser erste Teil sich vom oberen Ende der zweiten Zone bis zu einem Niveau derselben erstreckt, das zwischen ihrer halben Höhe und dem unteren Drittel ihrer gesamten Höhe liegt, sich an diesen ersten Teil der zweiten Zone ein zweiter Teil anschließt, der die Rolle der Reifungszone spielt, die in der Verlängerung des ersten Teils gelegen ist und in derem Inneren die Temperatur konstant zwischen 35 und 50°C, vorzugsweise zwischen 40 und 45°C, gehalten wird, das Gemisch den zweiten Teil der zweiten Zone in Form einer kristallisierten, an Kristallen reichen Masse verläßt, von der ausgehend man die letztgenannten gewinnt, wobei das Einleiten der Kristallisation im Bereich der ersten Zone durch das Recyclisieren im Bereich derselben einer Fraktion des die zweite Zone oder den ersten Teil der zweiten Zone durchlaufenden Gemischs unterstützt wird, wobei diese recyclisierte Fraktion 10 bis 120% der Menge des in die erste Zone eingebrachten Sirups entspricht, und diese Fraktion im in der unteren Hälfte der zweiten Zone oder in der unteren Hälfte des ersten Teils der zweiten Zone gelegenen Bereich entnommen wird.

2. Verfahren gemäß Anspruch 1, dadurch gekennzeichnet, daß der eingesetzte Gluconsäuresirup von Kristallen und Keimen frei ist.

3. Verfahren gemäß einem der Ansprüche 1 und 2, dadurch gekennzeichnet, daß der Gehalt an Gluconsäure des eingesetzten Sirups mehr als 90 Gew.-% beträgt.

4. Verfahren gemäß einem der Ansprüche 1 bis 3, dadurch gekennzeichnet, daß das die erste Zone verlassende Gemisch eine Konzentration an Kristallen von 3 ist 10 Gew.-% besitzt.

5. Verfahren gemäß einem der Ansprüche 1 bis 4, dadurch gekennzeichnet, daß die Achse der zweiten Zone in der Verlängerung derjenigen der ersten Zone liegt.

6. Verfahren gemäß einem der Ansprüche 1 bis 5, dadurch gekennzeichnet, daß der insgesamt abnehmende Temperaturgradient 0,3 bis 0,6°C/h beträgt.

7. Verfahren gemäß einem der Ansprüche 1 bis 6, dadurch gekennzeichnet, daß die recyclisierte Fraktion 40 bis 110%, vorzugsweise 80 bis 100%, der Menge des in die erste Zone eingebrachten Sirups beträgt.

8. Vefahren gemäß einem der Ansprüche 1 bis 7, dadurch gekennzeichnet, daß die recyclisierte Fraktion vor ihrem Einbringen in den Bereich des oberen Endes der ersten Zone einer Fragmentierung der Kristalle, die sie enthält unterzogen wird.

9. Vorrichtung für die kontinuierliche Kristallisation von Gluconodeltalacton, dadurch gekennzeichnet, daß sie im wesentlichen zwei Behälter mit vorzugsweise im wesentlichen vertikalen Achsen, die vorzugsweise übereinander gelegen sind, enthält, wobei die Achsen der beiden Behälter vorzugsweise im wesentlichen in Verlängerung zueinander vorliegen,
– wobei der erste Behälter oder der Behälter für das Einleiten der Kristallisation einesteils ausgestattet ist mit einem System für die Zufuhr von an Gluconsäure reichem Sirup in der Nähe seines oberen Endes, anderenteils mit einem System für das Rühren des Inhalts des Behälters und einem System zur Einstellung der Temperatur, dazu geeignet im Inneren des Behälters eine an sämtlichen Stellen im wesentlichen konstante Temperatur zu schaffen und schließlich mit einem Extraktionssystem, das in der Nähe seines unteren Endes gelegen ist, wobei dieses System befähigt ist, das im Inneren des Behälters gebildete Gemisch an Sirup und Kristallen zu entnehmen und dieses Gemisch einer Stelle zuzuführen, die gelegen ist in der Nähe des oberen Endes des
– zweiten oder eigentlichen Kristallisationsbehälters, der mit einem System zum Durchkneten des Inhalts und mit einem System zur Einstellung der Temperatur versehen ist, welches dazu geeignet ist im Inneren der der Kristallisation unterzogenen und ihn füllenden Masse einen insgesamt von oben nach unten ab dem oberen Ende des Behälters abnehmenden Temperaturgradienten zu schaffen, wobei dieser Gradient vorzugsweise auf einen ersten Teil dieses zweiten Behälters begrenzt ist, der sich bis zu einem zwischen der halben Höhe des Behälters gelegenen Bereich erstreckt, das System zur Einstellung der Temperatur in dem zweiten Teil dieses zweiten Behälters eine insgesamt konstante Temperatur hervorruft, die in der Nähe derjenigen im unteren Bereich des Gradienten vorherrschenden liegt, wobei der zweite Teil, der eine Reifungszone darstellt, sich nach unten dem ersten Teil anschließt, und wobei ferner der zweite Behälter weiterhin in der Nähe seines unteren Endes versehen ist mit einem System zur kontinuierlichen Entnahme eines mit GDL-Kristallen stark angereicherten Produkts, das mit geeigneten Mitteln zu einer der Gewinnung der GDL-Kristalle aus diesem Produkt angepaßten System transportiert wird, und die Vorrichtung außerdem versehen ist mit einem System zur Recyclisierung zu einer vorzugsweise in der Nähe des oberen Endes des ersten Behälters gelegenen Stelle eines Teils des Inhalts des zweiten Behälters, der entnommen wird in einem Bereich innerhalb des unteren Teils des zweiten Behälters oder, wenn er vorgesehen ist, in dem unteren Teil des ersten Teils des zweiten Behälters, wobei das Recyclisierungssystem vorteilhaft Mittel zur Fraktionierung der in der recyclisierten Masse enthaltenen Kristalle umfaßt.

10. Vorrichtung gemäß Anspruch 9, dadurch gekennzeichnet, daß sie einen einzigen Behälter aufweist, in dessen Innerem man mit Hilfe geeigneter Mittel die Zonen für die Einleitung der Kristallisation, für die eigentliche Kristallisation und für die Reifung verwirklicht.

11. Vorrichtung gemäß Anspruch 9, dadurch gekennzeichnet, daß sie außer dem ersten und zweiten Behälter einen dritten Behälter enthält, der die Reifungszone verkörpert und vorzugsweise in Verlängerung der beiden anderen Behälter gelegen ist und geeignete Mittel aufweist, um der Masse, die ihn durchläuft und dem zweiten Behälter entstammt, eine konstante Temperatur zu verleihen.

**Claims**

1. Process for the continuous crystallization of gluconodeltalactone characterized by the fact that a gluconic acid syrup of a richness in gluconic acid higher than 80%, of a concentration in dry matter of 80 to 95%, preferably of 85 to 90% by weight and of a temperature of 60 to 85°C, preferably of 65 to 75°C, is introduced into a first crystallisation zone of axis preferably substantially vertical which it is brought to traverse under stirring and within which it is maintained at a substantially constant temperature, less by 2 to 10°C, preferably by 3 to 5°C than the saturation temperature, due to which the starting of crystallization is produced which is manifested by the formation of a mixture of syrup and of GDL crystals, the average dwell time of a given fraction of the mixture inside the zone being from 10 to 30 hours, preferably from 15 to 25 hours, so that this mixture emerging from the zone shows a concentration of crystals of 2 to 15%, said mixture emerging from the first zone being brought to pass through, from top to bottom, under malaxation, a second crystallization zone of axis preferably substantially vertical, a temperature gradient globally decreasing, possibly modulated of 0.2 to 1°C/hour, being imposed inside the second zone on the mixture which passes through it, the said gradient being preferably limited to a first part of the second zone, this first part extending from the upper end of the second zone to a level of the latter situated between its mid-height and the lower third of tis total height, this first part of the second zone being followed by a second part situated in extension of the first and within which the temperature is maintained constant from 35 to 50°C, preferably from 40 to 45°C, the mixture emerging then from the second part of the second zone in the form of a crystalline mass rich in crystals and from which the latter are recovered, the starting of the crystallization inside the first zone being facilitated by the recycling to the level of the upper end of the latter by a fraction of the mixture traversing the second zone or the first part of the second zone, this recycled fraction representing from 10 to 120% of the amount of syrup introduced into the first zone, this fraction being taken up at the level situated in the lower half of the second zone or in the lower half of the first part of the second zone.

2. Process according to claim 1, characterized by the fact that the gluconic acid syrup therein employed is free from crystals and from nuclei.

3. Process according to one of claims 1 and 2, characterized by the fact that the richness in gluconic acid of the syrup used is higher than 90% by weight.

4. Process according to one of claims 1 to 3, characterized by the fact that the mixture emerging from the first zone has a concentration of crystals of 3 to 10% by weight.

5. Process according to one of claims 1 to 4, characterized by the fact that the axis of the second zone is situated in extensions of that of the first zone.

6. Process according to one of claims 1 to 5, characterized by the fact that the temperature gradient is decreasing overall by 0.3 to 0.6°C/hour.

7. Process according to one of claims 1 to 6, characterized by the fact that the recycled fraction represents from 40 to 110%, preferably from 80 to 100% of the amount of syrup introduced in the first zone.

8. Process according to one of claims 1 to 7, characterized by the fact that the recycled fraction is subjected to fragmentation of the crystals that it contains before its introduction at the level of the upper end of the first zone.

9. Installation for the continuous crystallization of gluconodeltalactone characterized by the fact that it comprises essentially two vessels of preferably substantially vertical axes arranged preferably one above the other, the axes of the two vessels being preferably substantially in extension of one another,

– the first vessel or crystallization starting vessel, being equipped on one hand with a system for feeding syrup rich in gluconic acid in the vicinity of its upper end, on the other hand with a stirring system of the contents of the vessel and with a system of regulation of temperature adapted to establish inside the vessel a temperature substantially constant at all points and finally with an extraction system arranged in the vicinity of its lower end, this system being adapted to extract the mixture of syrup and of crystals formed inside the vessel and to conduct this mixture to a point situated in the vicinity of the upper end of

– the second vessel, or crystallization vessel proper and equipped with a malaxation system of the contents and with a system for regulation of temperature adapted to establish within the mass subject to crystallization which fills it, a temperature gradient decreasing globally from top to bottom since the upper end of the vessel, this gradient being advantageously limited to a first part of the second vessel which extends to a level situated between the mid-height and the lower third of the total height of the vessel, the said temperature regulating system being adapted to establish in the second part of this second vessel, a temperature globally constant close to that existing at the level of the lower end of the abovesaid gradient, said second part which consitutes a ripening zone succeeding downwardly to the first part, said second vessel being furthermore equipped, close to its lower end, with a system for extracting continuously a product highly enriched in crystals of GDL which is led by suitable means to a system adapted to recover the GDL crystals from this product, said installation being in addition equipped with a system for recycling to a point situated preferably in the vicinity of the upper end of the first vessel of a part of the contents of the second vessel taken up at a level situated in the lower half of the second vessel and, if it exists, in the lower half of the first part of the second vessel, said recycling system comprising advantageously means for fractionating the crystals contained in the recycled mass.

10. Installation according to claim 9, characterized by the fact that it comprises a single vessel within which there is materialized by way of appropriated means the crystallization starting zone, the crystallization zone proper and the ripening zone.

11. Installation according to claim 9, characterized by the fact that it comprises, besides the first and second vessel, a third vessel materializing the ripening zone, preferably located in extension to the two other vessels and comprising means adapted to impose a temperature constant at all points to the mass by which it is traversed and which is coming from the second vessel.